## Europäisches Patentamt

## European Patent Office

## Office européen des brevets

(11) Veröffentlichungsnummer: **0 163 976**
**B1**

(12) **EUROPÄISCHE PATENTSCHRIFT**

(45) Veröffentlichungstag der Patentschrift:
**03.08.88**

(51) Int. Cl.⁴: **G 01 N 27/26**, G 01 N 27/46, G 01 N 27/30

(21) Anmeldenummer: **85105658.0**

(22) Anmeldetag: **08.05.85**

(54) **Messvorrichtung zur Bestimmung der Aktivität oder der Konzentration von Ionen in Lösungen.**

(30) Priorität: **08.05.84 DE 3416956**

(43) Veröffentlichungstag der Anmeldung:
**11.12.85 Patentblatt 85/50**

(45) Bekanntmachung des Hinweises auf die Patenterteilung:
**03.08.88 Patentblatt 88/31**

(84) Benannte Vertragsstaaten:
**DE FR GB IT**

(56) Entgegenhaltungen:
**AT - B - 377 366**
**DD - A - 132 210**
**DE - A - 2 726 772**
**DE - A - 2 908 719**
**DE - A - 3 206 049**
**US - A - 3 556 950**

(73) Patentinhaber: **Fresenius AG, Gluckensteinweg 5, D-6380 Bad Homburg v.d.H. (DE)**

(72) Erfinder: **Metzner, Klaus, Alolfstrasse 17b, D-6380 Bad Homburg (DE)**
Erfinder: **Westphal, Detlef, Dr., Starenweg 1, D-6370 Oberursel 4 (DE)**
Erfinder: **Allendörfer, Wolfgang, Louisenstrasse 87, D-6380 Bad Homburg (DE)**
Erfinder: **Hahnel, Reinhard, Heinrich-Schmidt-Strasse 16, D-6304 Lollar (DE)**
Erfinder: **Polaschegg, Hans-Dietrich, Dr., Grünwiesenweg 9, D-6370 Oberursel (DE)**

(74) Vertreter: **Görtz, Dr. Fuchs, Dr. Luderschmidt Patentanwälte, Sonnenberger Strasse 100 Postfach 26 26, D-6200 Wiesbaden (DE)**

ACTORUM AG

## Beschreibung

Die Erfindung betrifft eine Messvorrichtung zur Bestimmung der Aktivität oder der Konzentration von Ionen in Lösungen, mit wenigstens einer Messelektrode und einer Referenzelektrode, die mit einem Durchflusskanal miteinander verbunden sind, dessen erster Ausgang mit der Umgebung in Verbindung steht und dessen zweiter Ausgang durch eine erste Leitung, in die eine erste Pumpe und wenigstens ein Ventil eingeschaltet ist, mit wenigstens einem Kalibrierlösungsbehälter verbunden ist.

Eine Messvorrichtung der eingangs erwähnten Art ist beispielsweise aus den US-PS 4 206 027, 3 151 052 und 3 556 950 sowie aus der Monographie von Karl Cammann, «Das Arbeiten mit ionenselektiven Elektroden», Springer-Verlag 1977, S. 190–194, bekannt.

Jede dieser Vorrichtungen besteht aus einer Mehrzahl von Elektroden, die aneinander angeordnet sind und miteinander durch einen Durchflusskanal in Verbindung stehen. Sie bilden somit eine Durchflussanordnung, die auf der einen Seite über eine Leitung mit Behältern, die eine oder mehrere Standardlösungen und eine Messlösung aufweisen, und auf der anderen Seite mit einer Pumpe in Verbindung steht. Um die Behälter mit der Durchflussanordnung in Verbindung zu bringen, ist in der Verbindungsleitung zur Durchflussanordnung ein Mehrwegeventil vorgesehen, das durch ein Steuergerät entsprechend geöffnet oder geschlossen wird.

Eine derartige Durchflussanordnung kann mehrere unterschiedliche Elektroden, beispielsweise ionenselektive Elektroden aufweisen.

Mit einer derartigen Anordnung wird zunächst die Messung der Probe vorgenommen, an die sich die Kalibrierung anschliesst. Dabei wird zunächst eine erste Standardlösung über das Mehrwegeventil zur Zuführungsleitung zugeschaltet und dann durch die Pumpe in die Durchflussanordnung gepumpt und danach die Kalibrierung des gesamten Systems vorgenommen.

In gleicher Weise erfolgt die Kalibrierung einer zweiten oder weiteren Standardlösung, die bis zur nächsten Messung in der Anordnung verbleibt.

Eine derartige Kalibrierung und Messung ist bei der bekannten Vorrichtung zum einen sehr aufwendig und zum anderen mit erheblichen Fehlern behaftet. Dies ist beispielsweise darauf zurückzuführen, dass im Zuführungsschlauch zum Durchflusssystem und im Durchflusssystem selbst Reste der zuletzt bestimmten Flüssigkeit zurückbleiben, mit der Folge, dass die darauffolgende Flüssigkeit mit der zuvor gemessenen Flüssigkeit verschmutzt wird. Demzufolge muss also zunächst die gesamte Zuführungsleitung und das Durchflusssystem selbst solange mit der anderen Lösung gespült werden, bis die völlige Entfernung von Resten der zuvor gemessenen Lösung sichergestellt ist.

Eine derartige Verfahrensweise ist sowohl zeitaufwendig als auch materialaufwendig, so dass insgesamt gesehen der Betrieb einer derartigen Vorrichtung nachteilig ist.

Bei einer weiteren bekannten Vorrichtung ist die Pumpe stromauf der Durchflussvorrichtung in der Zuführungsleitung vorgesehen, während sich der Probenanschluss stromab der Durchflussanordnung befindet.

Bei dieser Vorrichtung werden die Kalibrierungslösungen durch Rechtslauf der Pumpe in die Durchflussanordnung gefördert, während die Probe durch Linkslauf der Pumpe in die Durchflussanordnung gezogen wird und aus dieser durch die Pumpe hindurch in den Abfall gefördert wird. Stromauf der Pumpe ist ebenso wie bei der vorstehend erläuterten Vorrichtung ein Mehrwegeventil bzw. eine Verzweigung von mehreren Leitungen vorgesehen, in die jeweils ein Ventil eingeschaltet ist.

Auch diese Anordnung weist den Nachteil auf, dass nach dem Absaugen der Probe in der Verbindungsleitung zwischen der Durchflussanordnung und dem Mehrfachventil Reste der Probe verbleiben, was bei der Zuführung der ersten Kalibrierlösung zu einer Vermischung der Kalibrierflüssigkeit mit den Proberesten im Schlauch und im Mehrfachventil führt.

Des weiteren besteht die Möglichkeit, dass das Mehrfachventil bzw. der Verteiler durch Reste der Probenflüssigkeit, beispielsweise Blut, dauerhaft verschmutzt wird, da sich das Blut festsetzt.

Der Erfindung liegt daher die Aufgabe zugrunde, die Messvorrichtung der eingangs erwähnten Art so fortzubilden, dass möglichst geringe Mengen von Standard- und Messlösung bei möglichst kurzem Zeitaufwand für eine genaue Messung notwendig sind.

Die Lösung der Aufgabe erfolgt durch die kennzeichnenden Merkmale des Anspruchs 1 oder 2.

Die erfindungsgemässe Messvorrichtung weist zunächst den Vorteil auf, dass die Vermischungsbereiche, d.h. die Bereiche, in denen es zwangsläufig zur Vermischung der Kalibrierlösungen und der Messlösung kommt, möglichst gering gehalten werden. Dies wird dadurch erreicht, dass in einer ersten Ausführungsform die Zuführungsleitung für die Kalibrierlösungen benachbart zum Eingang der Messvorrichtung eine abzweigende Leitung aufweist, die zum Abführen der gemessenen Flüssigkeit (Kalibrierlösung oder Messlösung) dient. Demzufolge kommt die gesamte Zuführungsleitung nicht mehr in Kontakt mit der zu messenden Lösung.

Der Bereich, den sämtliche Flüssigkeiten durchströmen müssen, bleibt also im wesentlichen auf den Durchströmungskanal der Messanordnung selbst beschränkt.

Weiterhin ist in der Abzweigungsleitung eine Pumpe vorgesehen, mit der die abzupumpende Flüssigkeit durch die Messvorrichtung und die Abzweigungsleitung gefördert wird. Diese Pumpe soll wenigstens das gleiche Fördervermögen wie die in der Zuführungsleitung angeordnete Pumpe aufweisen.

Vorteilhafterweise wird eine einzige Pumpe in Form einer peristaltischen Rollenpumpe verwendet, die beidseitig wirkt, d.h. sowohl die Zuführungsleitung als auch die Abführungsleitung sind jeweils in ein Pumpensegment der Rollenschlauchpumpe eingelegt. Durch entsprechende Wahl des Innendurchmessers der Schläuche können gleiche oder veränderliche Fördervolumina bestimmt werden. Vorzugsweise weist der Zuführungsschlauch einen geringeren Innendurchmesser auf als der Abführungsschlauch, d.h. das Fördervolumen des Abführungsschlauchs ist grösser als das des Zuführungsschlauchs.

Des weiteren kann bei der ersten Ausführungsform verhindert werden, dass eine verunreinigte Kalibrierlösung in die Durchflussanordnung einströmen kann. Dies wird dadurch erreicht, dass die beidseitig wirkende Pumpe die Kalibrierlösung bis zum Verzweigungspunkt pumpt. Infolge der Pumpwirkung des anderen Pumpensegments wird die zugeführte Kalibrierlösung durch die Abzweigungsleitung abgepumpt, so dass hierdurch die Kalibrierlösung an der Durchflussanordnung vorbei in den Abfluss gepumpt wird. Diese Pumpweise erfolgt solange, bis evtl. im Zuführungszweig vorhandene grobe Verunreinigungen entfernt sind. Anschliessend wird ein stromab des Pumpsegments im Abflusszweig angeordnetes Ventil geschlossen, was zur Folge hat, dass dieses Pumpsegment nicht mehr weiterfördert. Hierdurch erfolgt zwangsläufig das Einpumpen der reinen Kalibrierlösung in die Durchflussanordnung, da das eine Pumpensegment in der Zuführungsleitung weiterfördert.

Die Förderung der Kalibrierlösung erfolgt solange, bis der Durchflusskanal in der Messvorrichtung vollständig gefüllt ist. Anschliessend wird das Ventil geschlossen, das ausgangs des Behälters mit der Kalibrierlösung angeordnet ist.

Vorteilhafterweise bleibt die Rollenschlauchpumpe nach dem Schliessen dieses Ventils in Betrieb und erzeugt durch die ständige Okklusion des Schlauchs ein Pulsieren der im Schlauch und in der Durchflussanordnung befindlichen Flüssigkeitssäule. Hierdurch werden insbesondere in der Durchflussanordnung sämtliche zurückgebliebenen groben Verschmutzungen, also auch diejenigen Probenbestandteile, die an der Wand des Durchflusskanals hängenblieben, durch die Kalibrierlösung aufgenommen und in dieser verteilt.

Wird nun das in der Abflussleitung befindliche Ventil wieder geöffnet, so wird ein Unterdruck in der gesamten Durchflussanordnung erzeugt, was zur Folge hat, dass die im gesamten Durchflusskanal befindliche Flüssigkeitssäule in den Abfluss gefördert wird. Dabei sind die in der Zuführungsleitung vorgesehenen Ventile geschlossen. Danach wird das Abflussventil geschlossen und das stromab des Behälters mit der Kalibrierlösung befindliche Ventil wieder geöffnet, wodurch erneut reine Kalibrierlösung in den Durchflusskanal einströmt. Bei diesem Schritt befindet sich also innerhalb der Messgrenzen reine Kalibrierlösung in der Durchflussanordnung, so dass die Kalibrierung dieser Durchflussanordnung nunmehr erfolgen kann.

Nach der Kalibrierung mit einer ersten Kalibrierlösung werden die vorstehenden Schritte zur Kalibrierung mit einer zweiten oder weiteren Kalibrierlösung wiederholt.

Nach Abschluss der Kalibrierung erfolgt die Feststellung des Messwerts. Danach schliesst sich ein neuer Messzyklus an, bestehend aus Einführung der Messlösung in die Messvorrichtung und Kalibrierung. Die Zuführung der Messlösung erfolgt von der anderen Öffnung des Zuführungskanals durch eine Kapillare, wobei die Pumpe auf der Seite der Abflussleitung weiterbetrieben wird, in der das dort befindliche Ventil geöffnet ist. Nach der Füllung des Kanals erfolgt die Messung der Parameter mit Hilfe der Elektroden und der nachgeschalteten Auswerteeinheit.

Nach der Messung erfolgt dann wiederum die Kalibrierung mit den Kalibrierungslösungen, wie dies vorstehend beschrieben worden ist.

In einer weiteren zweckmässigen Ausführungsform ist auf der Zuführseite der Kalibrierungslösungen eine weitere Leitung vorgesehen, in die ebenfalls ein Ventil eingeschaltet ist. Durch diese Leitung kann Luft zugeführt werden, und diese Luftzuführung hat den Vorteil, dass jeweils nur eine bestimmte Flüssigkeitsmenge durch die Zuführungsleitung gepumpt werden kann, ohne dass es beim Abschliessen des Ventils, das dem jeweiligen Kalibrierlösungsbehälter zugeordnet ist, zu einem Stillstand der geförderten Flüssigkeitssäule infolge Unterdruck kommt.

Demzufolge kann also bei dieser Ausführungsform eine erhebliche Menge an Flüssigkeit durch Belüftung des Systems eingespart werden.

In einer zweiten bevorzugten Ausführungsform der erfindungsgemässen Messvorrichtung erfolgt die Förderung der Flüssigkeiten durch den Durchströmungskanal nur in einer Richtung. Bei dieser Ausführungsform wird das eine Ende des Durchströmungskanals der erfindungsgemässen Messvorrichtung mit einer Zuführungsleitung verbunden, von der jeweils Leitungen abzweigen, die mit Kalibrierlösungsbehältern bzw. mit der Umgebung (Luft) verbunden sind. Diese abzweigenden Leitungen können durch entsprechende Ventile abgesperrt werden. Stromab dieser Ventile ist in diese Leitung wiederum eine Pumpe eingeschaltet, über die die zu fördernden Fluide in den Messkanal gefördert werden.

Auf der anderen Seite des Messkanals mündet die abführende Leitung in ein Auffanggefäss, so dass die Druckschwankungen im Kanal reduziert sind. Dieses Auffanggefäss ist mit einer Abflussleitung in Verbindung, in die eine Pumpe eingeschaltet ist. Vorteilhafterweise ist diese Pumpe in Verbindung mit der Pumpe in der Zuführungsleitung zu einer beidseitig wirkenden Pumpe zusammengefasst, wie dies vorstehend bei der ersten Ausführungsform erläutert ist.

Die Zuführung der Messlösung erfolgt auf der Seite der Zuführungsleitung unmittelbar in den Messkanal dadurch, dass eine Kanüle durch ein vor dem Messkanal angeordnetes Septum durchgestochen und in den Messkanal eingeführt wird. Diese Anordnung ist insbesondere bei Blutentnahmen durch eine Spritze vorteilhaft, die dann in den Messkanal unmittelbar eingeführt werden kann.

Weitere Merkmale, Vorteile und Ausführungsformen der Erfindung sind in der nachfolgenden Beschreibung unter Bezugnahme auf die Zeichnung erläutert. Es zeigen:

Fig. 1 schematisch die erfindungsgemässe Messvorrichtung gemäss einer ersten Ausführungsform,

Fig. 2 schematisch die erfindungsgemässe Messvorrichtung gemäss einer zweiten Ausführungsform,

Fig. 3 einen Schnitt durch die Lichtschrankenanordnung gemäss Fig. 1 entlang der Linie III–III,

Fig. 4 eine Draufsicht auf den Elektrodenblock gemäss Fig. 1 oder 2,

Fig. 5 eine Seitenansicht des Elektrodenblocks,

Fig. 6 eine Seitenansicht einer Einzelelektrode,

Fig. 7 eine Draufsicht auf die Referenzelektrode, die im Elektrodenblock eingebaut ist,

Fig. 8 eine Frontansicht der Referenzelektrode,

Fig. 9 einen Schnitt durch die Referenzelektrode gemäss Fig. 7 entlang der Linie IX–IX und

Fig. 10 einen vergrösserten Querschnitt durch einen Teil der Referenzelektrode gemäss Fig. 8.

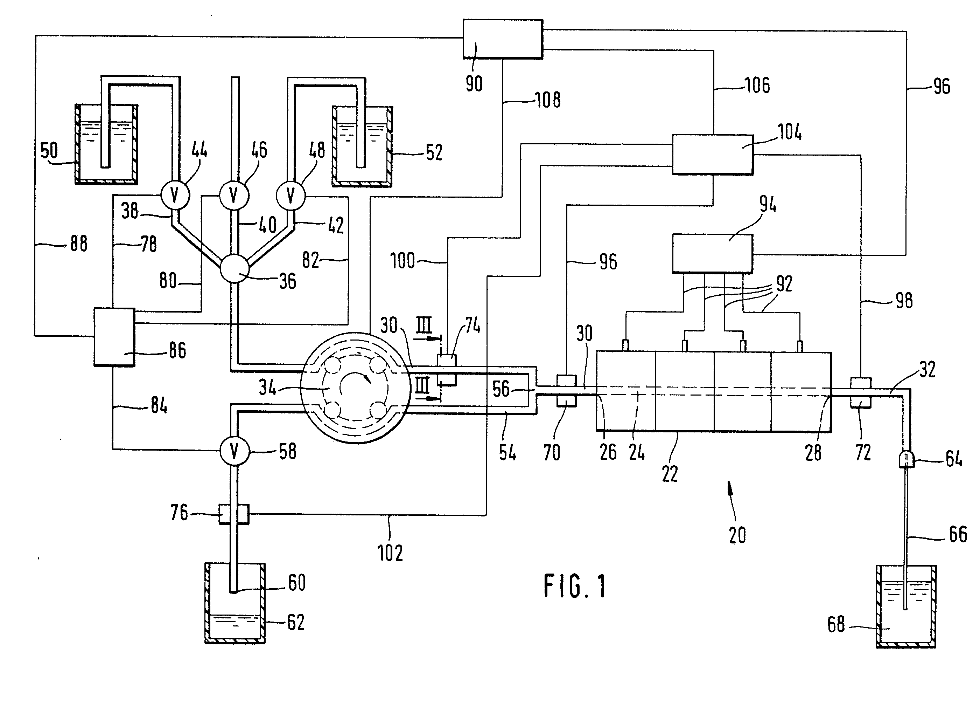

In Fig. 1 ist ein Messgerät 20 gezeigt, das die erste bevorzugte Ausführungsform der Erfindung darstellt.

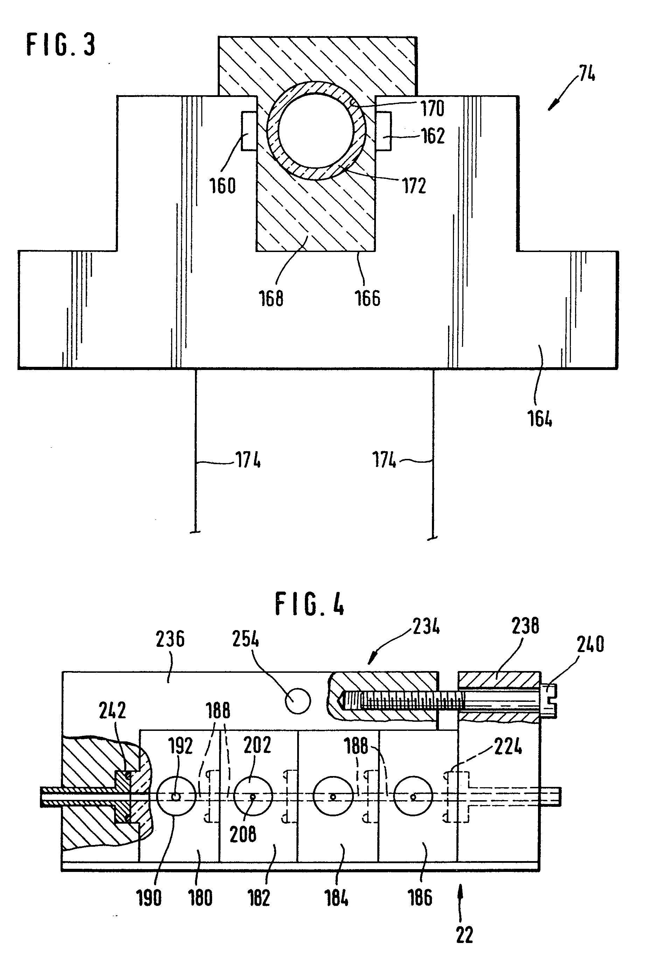

Dieses Messgerät 20 weist einen Elektrodenblock 22 auf, der im Beispiel 2 aus vier Einzelelektroden zusammengesetzt ist. Ein derartiger Elektrodenblock wird nachstehend, wie in Fig. 4 gezeigt, erläutert.

Dieser Elektrodenblock 22 ist mit einem Durchflusskanal 24 durchsetzt, wobei sämtliche Elektroden jeweils einen Teil dieses Durchflusskanals aufweisen. Beim Zusammensetzen der Elektroden fluchten diese Teile unter axialer Ausrichtung und bilden somit den Durchflusskanal 24.

Der Durchflusskanal 24 weist im Elektrodenblock zwei Endbereiche 26 und 28 auf, die jeweils mit abzweigenden Leitungen 30 und 32 verbunden sind.

Die Leitung 30 erstreckt sich also von dem Endbereich 26 weg und führt durch eine Pumpe 34, die vorteilhafterweise als peristaltische Rollenschlauchpumpe ausgebildet ist, wobei sie in ein Segment, wie in Abb. 1 gezeigt, eingelegt ist. Stromauf der Pumpe weist die Leitung 30 einen Verzweigungs- oder Mischpunkt 36 auf, von dem mehrere Leitungen abzweigen, nämlich die Leitung 38, 40 und 42. In diese Leitungen 38–42 ist jeweils ein Absperrorgan 44, 46 und 48 eingeschaltet.

Das Ende der Leitung 38 ist mit einem Gefäss 50 für eine erste Kalibrierlösung verbunden, während die Leitung 42 mit einem Gefäss 52 mit einer zweiten Kalibrierlösung verbunden ist. Die Leitung 40 stellt einen Luftanschluss dar, steht also mit der Umgebung durckausgeglichen in Verbindung.

Zwischen der Pumpe 34 und dem Endbereich 26 des Elektrodenblocks 2 zweigt von der Leitung 30 eine Abflussleitung 54 am Verzweigungspunkt 56 ab. Diese Leitung 54 ist ebenfalls wie die Leitung 30 in die Pumpe 34 eingelegt, so dass auch hier wiederum ein Pumpsegment der Pumpe 34 auf die Leitung 54 einwirken kann. Somit wirkt also die Pumpe 34, die vorteilhafterweise als peristaltische Schlauchpumpe ausgebildet ist, beidseitig. Natürlich kann auch diese beidseitige Pumpe 34 durch zwei Pumpen ersetzt werden, wobei jede auf die Leitung 30 bzw. 54 einwirkt.

Da das Fördervermögen der vorteilhafterweise als Schläuche ausgestalteten Leitungen 30 und 54 beim Einsatz einer peristaltischen Schlauchpumpe 34 vom Innenquerschnitt des eingesetzten Schlauchs abhängt, ist es bevorzugt, dass die Leitung 54 wenigstens das gleiche Fördervolumen wie die Leitung 30 zulässt. Insbesondere ist das Fördervolumen der Leitung 54 grösser als das der Leitung 30, d.h. der Innenquerschnitt der Leitung 54 ist grösser als derjenige der Leitung 30. In ähnlicher Weise ist das Fördervolumen von separaten Pumpen derart ausgestaltet, dass die mit der Leitung 54 in Verbindung stehende Pumpe wenigstens das gleiche Fördervolumen besitzt wie die in die Leitung 30 eingesetzte Pumpe. Vorzugsweise ist das Fördervolumen beider Pumpen gleich.

Stromab der Pumpe 34 ist in die Leitung 54 ein Ventil 58 eingeschaltet.

Das Leitungsende 60 der Leitung 54 mündet in einem Abfallbehälter 62, der zur Aufnahme der verbrauchten Flüssigkeiten dient.

Der andere Endbereich 28 des Elektrodenblocks 2 ist – wie bereits vorstehend erwähnt – über eine Leitung 32 mit einem Adapter 64 verbunden, der so ausgelegt ist, dass er eine auswechselbare Aspirationskanüle 66 aufnehmen kann.

Vorzugsweise ist sowohl die Leitung 32 als auch die Aspirationskanüle 66 flexibel ausgestaltet, so dass diese gesamte Anordnung in beliebiger Weise angeordnet werden kann. Zweckmässigerweise wird die Aspirationskanüle 66 in Form eines flexiblen Kunststoffrohres eingesetzt, das nach einmaligem Gebrauch wieder ausgewechselt werden kann.

Andererseits kann jedoch aber auch in den Adapter 64 die Kanüle einer Spritze eingesteckt werden, die beispielsweise für die Blutabnahme eingesetzt worden ist.

Wie in Fig. 1 gezeigt, kann die Aspirationskanüle 66 in einen Behälter 68 eintauchen, der die der Messung zu unterwerfende Flüssigkeit enthält.

Benachbart zu den Endbereichen 26 und 28 weisen die Leitungen 30 und 32 jeweils Sensoren 70 und 72 auf, mit denen die durch die Leitungen 30 und 32 ankommenden Flüssigkeitssäulen sicher in ihrer Positionierung erkannt und überwacht werden können. Vorteilhafterweise werden als Sensoren 70 und 72 IR-Lichtschranken eingesetzt.

Ein weiterer derartiger Sensor 74 ist in der Leitung 30 zwischen der Pumpe 34 und dem Verzweigungspunkt 56 angeordnet. Schliesslich befindet sich noch ein Sensor 76 im Bereich der Leitung 54 zwischen dem Ventil 58 und dem Leitungsende 60.

Auch die letztgenannten Sensoren sind vorteilhafterweise als IR-Lichtschranken ausgebildet.

Mit den Sensoren 70 und 72 wird die korrekte Füllung der Messkapillaren, der Aspirationskanüle, der Leitung 32, des Durchflusskanals 24 und der Leitung 30, gesteuert.

Der Sensor 74 dient als Monitor für die Zufuhr der Kalibrierlösungen aus den Behältern 50 und 52 bzw. der Luft.

Der Sensor 76 kontrolliert vorteilhafterweise die Abflussleitung 54 und überprüft durch Rückwärtspumpen der Pumpe, also durch die Änderung der Drehrichtung der Pumpe, ob der Abfallbehälter 62 gefüllt ist. Zu diesem Zweck ist das Leitungsende 60 der Leitung 54 nur kurz unterhalb des oberen Randes des Abfallbehälters 62 angeordnet. Sofern Abfallflüssigkeit dieses Leitungsende 60 erreicht, kommt es zu einem Signal am Sensor 76 beim Hochziehen einer entsprechenden Flüssigkeitssäule.

Wie aus Fig. 1 ersichtlich, sind die Ventile 44, 46, 48 und 58 über die Leitungen 78–84 mit einem Ventilsteuergerät 86 verbunden, das über eine Leitung 88 mit einer allgemeinen Steuervorrichtung 90 verbunden ist.

Die Elektroden des Elektrodenblocks 22 sind über Leitungen 92 mit einem Elektrodenanschlussgerät 94 verbunden, das über eine Leitung 96 ebenfalls mit der Steuervorrichtung 90 verbunden ist.

Schliesslich sind die Sensoren 70, 72, 74 und 76 über Leitungen 98–102 mit einem Sensorsteuergerät 104 verbunden, das über die Leitung 106 mit der Steuervorrichtung 90 verbunden ist.

Weiterhin ist die Steuervorrichtung 90 über die Leitung 108 mit dem Motor 34 verbunden.

Natürlich können auch die Geräte 86, 90, 94 und 104 zu einem einzigen Gerät integriert sein, was vorteilhafterweise der Fall ist.

Über das Ventilsteuergerät 86 werden die Ventile 44–48 bzw. 58 auf Befehl der Steuervorrichtung 90 geöffnet oder geschlossen. Diese Steuervorrichtung nimmt ebenfalls das Signal aus dem Sensoranzeigegerät 104 entgegen und wertet dies in entsprechender Weise aus, wobei das Ventilsteuergerät 86 bzw. der Motor 34 über die Leitung 108 entsprechend angesteuert werden.

Schliesslich nimmt das Steuergerät 90 auch die Signale aus dem Elektrodenanschlussgerät 94 entgegen, das die Elektrodensignale umwandelt und auswertet.

Das in Fig. 1 gezeigte Messgerät 20 arbeitet auf folgende Weise:

Der Einsatz von ionenselektiven Durchflusselektroden im Elektrodenblock 22 zur Bestimmung der Aktivität bzw. der Konzentration bestimmter Ionenarten in Lösungen erfordert es, dass nach jeder Probenmessung eine Kalibrierung der Elektroden mit Lösungen genau bekannter Zusammensetzung durchgeführt wird. Dazu müssen die Kalibrierflüssigkeiten nach dem Absaugen der Probe jeweils in den Elektrodenkanal gepumpt werden, wobei zu vermeiden ist, dass es zu einer Vermischung von Probenresten und Kalibrierflüssigkeiten kommen kann.

Diese Forderung wird von dem Messgerät 20 erfüllt.

Für eine Probenaspiration über Aspirationskanüle 66 wird die Probe aus dem Behälter 68 mit Hilfe der Pumpe 34 bei geöffnetem Ventil 58 bis zur Verzweigung 56 angesaugt, wobei die Sensoren 70 und 72 die korrekte Füllung des Durchflusskanals 24 überwachen. Erreicht die Flüssigkeitssäule den Sensor 70, so wird die Pumpe noch für ein bestimmtes Zeitintervall weiterbetätigt, um sicherzustellen, dass Rückstände der Kalibrierlösungen im Durchflusskanal 24 völlig aus dem Messbereich in die Abfalleitung entfernt sind. Vorteilhafterweise wird die Probe nach der Eingabe um die Länge der Aspirationskanüle 66 weitertransportiert. Dadurch wird die Aspirationskanüle 66 frei und ein Abtropfen der Probe verhindert. Gleichzeitig wird der durch die Restflüssigkeit in der Messkapillare oder dem Durchflusskanal 24 besonders kontaminierte erste Teil der Probe aus dem Elektrodenblock 22 herausgeschoben und so die Probenverschleppung ohne Vergrösserung der Probemenge reduziert.

Hierauf erfolgt die Messung der gewünschten Parameter, wobei die Pumpe 34 zu diesem Zweck von der Steuervorrichtung 90 stillgesetzt wird.

Nach Abschluss der Messung der gewünschten Parameter, die auf dem Elektrodenanschlussgerät 94 vorteilhafterweise angezeigt werden, wird die Pumpe 34 solange bei geöffnetem Ventil 58 in Betrieb genommen, bis die Probe vollständig durch die Abflussleitung 54 in den Abfallbehälter 62 gepumpt worden ist.

Anschliessend erfolgt vorteilhafterweise eine Zwei-Punkt-Kalibrierung nach jeder Messung, so dass für jede Umgebungsbedingung die aktuelle Steilheit der Elektroden berücksichtigt werden kann.

Andererseits kann jedoch aber auch eine Ein-Punkt-Kalibrierung durchgeführt werden, sofern der hierdurch auftretende Fehler abgeschätzt werden kann.

Bei der Zwei-Punkt-Kalibrierung entfällt jedoch das beim bisherigen Verfahren erforderliche Nachkalibrieren nach einer bestimmten Zeit bzw. Anzahl von Messungen und die Möglichkeit von zunehmenden Fehlern beim Unterlassen dieser Massnahme. Sie setzt lediglich die gleiche Temperatur von Probe und Kalibrierflüssigkeiten voraus, was üblicherweise gewährleistet werden kann, da sich das Messgerät 20 üblicherweise bei der Umgebungstemperatur befindet und eine im Vergleich zur Probe (typisch wenige 100 µl) erheblich grössere Wärmekapazität aufweist. So nimmt beispielsweise eine Blutprobe von 37°C innerhalb von Sekunden die Umgebungstemperatur an.

Die Leitfähigkeit und die ionischen Aktivitätskoeffizienten besitzen ebenfalls eine deutliche Temperaturabhängigkeit. Üblicherweise werden diese Grössen bei einer zu bestimmenden Umgebungstemperatur gemessen und numerisch bzw. schaltungstechnisch auf den Wert von 25°C umgeformt.

Bei der hier vorteilhafterweise verwendeten Zwei-Punkt-Kalibrierung ist eine solche Temperaturkompensation im wesentlichen nicht notwendig. Wenn Probe und Kalibrierflüssigkeiten hinreichend genau übereinstimmende Temperaturen und Temperaturkoeffizienten haben, genügt die exakte Bestimmung von Aktivität und Leitfähigkeit der Kalibrierflüssigkeiten bei einer bestimmten Temperatur, beispielsweise bei 25°C.

Dabei werden die Spannungen der Elektroden bei vorgelegter Probe bei einer beliebigen Temperatur mit denen der Kalibrierflüssigkeiten verglichen. Den 25°C-Probenleitwert erhält man durch lineare Interpolation zwischen den Eckwerten der Kalibrierlösungen:

$$LF (Probe) = LF (1) + [LF (2) - LF (2)] \times [U(P) - U(1)]/[U(2) - U(1)]$$

Analoges gilt für die Aktivität A, wobei zu berücksichtigen ist, dass

$$dU = S(T) \times lg \, dA + dU (Stör)$$

gilt. (S: Steilheit, T: Temperatur, LF (1) bzw. (2): Leitfähigkeitswert für Lösung 1 bzw. 2; U: Potential; P: Probe)

Bei diesem relativen Verfahren geht im übrigen die Geometrie der LF-Messzelle ebenfalls nicht ein.

Nach dem Absaugen der Probe wird das Ventil 44 geöffnet, so dass die erste Kalibrierflüssigkeit durch die Leitung 38 in die Leitung 30 bis zum Verzweigungspunkt 56 und von dort durch den im anderen Pumpsegment erzeugten Unterdruck in die Leitung 54 weitergepumpt wird. Somit erfolgt also die Reinigung der Leitung 30 von Resten der zuvor übriggebliebenen Kalibrierflüssigkeit.

Nach einem bestimmten Zeitintervall wird das Ventil 58 in der Abflussleitung 54 geschlossen, was den Eintritt der Kalibrierlösung in den Durchflusskanal 24 bewirkt. Die Wirkung der Pumpe wird erst dann unterbrochen, wenn die Flüssigkeitssäule der ersten Kalibrierflüssigkeit durch den gesamten Durchflusskanal 24 hindurch gepumpt worden ist und den Sensor 72 erreicht hat, der ein entsprechendes Signal zum Schliessen des Ventils 44 an das Steuergerät 90 abgibt. Die Pumpe 34 pumpt jedoch weiter und induziert aufgrund ihres peristaltischen Charakters ein Pulsieren in der Schlauchleitung, so dass die gesamte Flüssigkeitssäule intensiv auch die Oberflächen benetzt, auf denen sich üblicherweise Probereste befinden. Weiterhin beginnen die Elektroden daher schon zu diesem Zeitpunkt sich auf die neue Lösung einzuschwingen.

Nach einer bestimmten Zeit wird das Ventil 58 in der Abflussleitung erneut geöffnet, so dass die im Durchflusskanal 24 befindliche Flüssigkeitsmenge wieder abgesaugt wird. Hierauf erfolgt das Schliessen des Ventils 58 und das erneute Öffnen des Ventils 44, um erneut frische Kalibrierlösung in den Durchflusskanal 24 einzuführen. Nach dem Haltesignal durch den Sensor 72 erfolgt dann die eigentliche Messung durch die Elektroden des Elektrodenblocks 22.

Das für die erste Kalibrierflüssigkeit beschriebene Verfahren wird identisch auch bei der zweiten Kalibrierflüssigkeit appliziert.

Für den Messfehler durch Probenverschleppung gilt folgendes:

Wenn v der Volumenanteil der Restprobe in dem Durchflusskanal (v: wenige Prozent) ist, dann kann der Beitrag der Probenverschleppung nach der Zwischenspülung mit v×v (unter 1‰) abgeschätzt werden. Somit ist die Kalibrierlösung innerhalb der Messgenauigkeit unverfälscht.

Weiterhin kommen durch die Zwischenspülung die Elektroden schon vor der Messung mit der zu messenden Lösung in Kontakt. Dadurch vergrössert sich die Zeit, die den Elektroden zum Einstellen auf die neue Messlösung zur Verfügung steht bzw. verringert sich der Bedarf an Messzeit.

Die beim Kalibrieren festgestellten Werte werden mit den Werten verglichen, die zuvor im Elektrodenanschlussgerät 94 gespeichert worden sind, und ggf. auf diese Werte bezogen. Hierauf erfolgt die eigentliche Messung der Probe, an die sich erneut eine Zwei-Punkt-Kalibrierung wieder anschliesst.

Für die nächste Messung einer Probe wird die in einem Siliconschlauch (Leitung 32) oder dem Adapter 64 gehaltene, flexible Kunststoffkanüle ausgewechselt. Somit entfällt ein Beitrag dieser Kanüle zur Probenverschleppung, was bei einer fixen Kanüle der Fall ist.

Bevorzugt wird jedoch nicht die gesamte Leitung 30 mit Kalibrierflüssigkeit gefüllt. Zu diesem Zweck werden die Ventile 44 bzw. 48 nur solange geöffnet, bis die zur Füllung des Durchflusskanals 24 notwendige Flüssigkeitsmenge aus dem Behälter 50 bzw. 52 abgezogen worden ist. Anschliessend werden die Ventile 44 bzw. 48 geschlossen und das Ventil 46 geöffnet, so dass Luft zutreten kann, d.h. kein Unterdruck in der Leitung 30 auftreten kann, der ansonsten die Flüssigkeitssäule in der Leitung 30 zurückhalten würde. Dieses Ventil 46 wird dann geschlossen, wenn die Flüssigkeitssäule den Sensor 72 erreicht hat. Ansonsten gleicht das weitere Vorgehen der vorstehend beschriebenen Verfahrensweise.

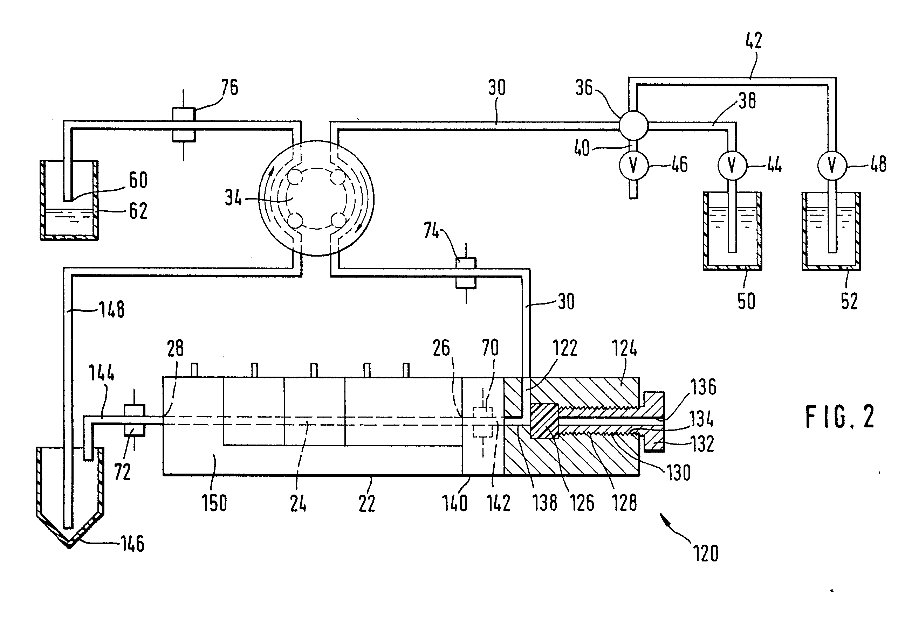

In Fig. 2 ist ein Messgerät 120 gezeigt, das die zweite Ausführungsform des erfindungsgemässen Messgerätes darstellt. Wie aus Fig. 2 ersichtlich ist, wurden für gleiche Teile die gleichen Bezugszeichen wie in Fig. 1 verwendet.

Demgemäss weist also das Messgerät 120 wiederum einen Elektrodenblock 22 auf, der mit einem Durchflusskanal 24 durchsetzt ist. Der Durchflusskanal steht wiederum mit der Zuführungsleitung 30 in Verbindung, in die der Sensor 74, ein Segment der Pumpe 34, der Mischpunkt 36, die Leitungen 38, 40 und 42, die Ventile 44, 46 und 48 und die Behälter 50 und 52 eingeschaltet sind. Allerdings mündet die Leitung 30 nicht unmittelbar in den Endbereich 26 des Durchflusskanals 24, sondern steht vielmehr mit einem Kanal 122 in Verbindung, dessen Achse im wesentlichen senkrecht auf der Achse des Durchflusskanals 24 steht und der mit diesem Durchflusskanal 24 in Strömungsverbindung steht. Dieser Kanal 122 ist in einem Septumadapter 124 angeordnet, der ein mit einer Kanülenspitze durchstechbares Septum 126 aus Gummi oder einem Kunststoff aufweist. Das Septum ist in einer entsprechenden Bohrung 128 im Septumadapter angeordnet, dessen Innenoberfläche zum Teil mit einem Gewinde 130 versehen ist. In dieses Gewinde lässt sich ein mit einem entsprechenden Gewinde 134 versehener Stopfen 132 einschrauben, der das Septum lagefixiert. Dieser Stopfen 132 weist einen zentralen Kanal 136 auf, der mit dem Durchströmungskanal 24 fluchtet und in seinem Durchmesser in etwa dem Durchmesser des Durchströmungskanals entspricht. Dieser Kanal 136 dient als Einführungshilfe für eine Injektionsnadel oder die Spitze einer Kanüle, die durch das Septum in den Kanal 24 eingeführt wird. Zu diesem Zweck ist der Spitzenadapter weiterhin mit einem Kanalstück 138 versehen, das im rechten Winkel von dem Kanal 122 abzweigt und mit dem Durchströmungskanal 24 fluchtet.

Weiterhin ist zwischen dem Elektrodenblock 22 und dem Septumadapter 124 ein Sensorgehäuse 140 angeordnet, das den Sensor 70 aufnimmt und ebenfalls mit einem Kanalstück 142 durchsetzt ist, das wiederum mit dem Durchflusskanal 24 fluchtet.

Der andere Ausgang oder Endbereich 28 ist mit einer Leitung 144 verbunden, die mit dem Sensor 72 versehen ist. Diese Leitung 144 mündet in einem Druckausgleichsgefäss 146, das vorteilhafterweise spitz im Bodenbereich zuläuft.

Bis zum Boden ist die Absaugleitung 148 geführt, die in das andere Segment der Pumpe 34 eingelegt ist und die, wie die Leitung 54 gemäss Fig. 1, im Abfallbehälter 62 endet. Weiterhin ist in dieser Absaugleitung 148 wiederum ein Sensor 76 angeordnet, der im Beispielsfall stromab der Pumpe 34 entsprechend der in Fig. 1 gezeigten Ausführungsform angeordnet ist.

Wie in Fig. 2 gezeigt, endet die Leitung 144 unter Druckausgleich vorteilhafterweise benachbart zum Endbereich 28 des Elektrodenblocks 22, wobei dieser Endbereich vorteilhafterweise an der Referenzelektrode 150 des Elektrodenblocks 22 gebildet ist. Eine derartige Anordnung ist deshalb von Vorteil, weil Druckschwankungen an der Referenzelektrode 150 so gering wie möglich zu halten sind. Infolge dessen befindet sich der Druckausgleichsbehälter 146 in unmittelbarer Nähe der Referenzelektrode 150 und das Ende des Schlauchs 144 auf Atmosphärendruck. Insofern ergeben sich an der Membran der Referenzelektrode 150 nur geringe Druckschwankungen um diesen Wert herum, so dass eine Infusion in die Referenzelektrode und damit Potentialsprünge u. dgl. verhindert werden. Eine derartige Infusion wäre durch den Überdruck in der Elektrolytleitung zu befürchten gewesen, der durch ein Schieben von Flüssigkeitssegmenten durch die Leitung entsteht. Dieser wächst mit der Länge der Leitung und der Zahl der Flüssigkeitssegmente in der Leitung. Erfindungsgemäss wird eine darauf zurückzuführende Drift der Referenzelektrode durch die Anordnung eines Druckausgleichsgefässes 146 vermieden.

Weiterhin ist die Schlauchgrösse der Leitung 148 so dimensioniert, dass die Förderleitung in der Abfall-Leitung 148 grösser ist als in der Leitung 144 bzw. dem Durchflusskanal 24. Auf diese Weise wird verhindert, dass der Druckausgleichsbehälter 146 überfüllt wird. Dieser besteht vorteilhafterweise aus einem Kunststoffmaterial und ist als Disposable konzipiert, so dass er bei Störungen ausgewechselt werden kann. Weiterhin treten für wenigstens einen Monat weder Sedimentation noch Keimbildung im Druckausgleichsbehälter 146 auf, wenn die Abfall-Leitung 148 mit einer starren Kunststoffkapillare bis in die Spitze des Behälters 146 geführt wird.

Wie aus der Ausführungsform gem. Fig. 2 ersichtlich, fehlt in der Abfall-Leitung 148 das Ventil 58 gem. Fig. 1.

Im übrigen ist die elektrische Verbindung der einzelnen Teile des Messgeräts 120 identisch mit der Ausführungsform gem. Fig. 1.

Wie aus Fig. 2 ersichtlich, ändert sich bei einer Probeneingabe mittels Spritze das Messverfahren auf folgende Weise:

Die Flüssigkeitssäule wird nur noch in einer Richtung durch den Durchflusskanal 24 geschoben. Beim Start wird zunächst die Benetzungsflüssigkeit (Kalibrierungslösung 2) aus den Kanälen 138, 142 und 24 dadurch gefördert, dass das Luftventil 46 geöffnet wird. Die Luft wird mit der Pumpe 34 durch die Leitung 30 sowie die Kanäle 122, 138, 142 und 24 sowie die Leitung 144 gepumpt und schiebt dabei die Flüssigkeitssäule vor sich her. Diese gelangt in den Druckausgleichsbehälter 146 und wird aus diesem über den separaten Pumpkreis, der durch die Leitung 148 gebildet wird, in den Abfallbehälter 62 gepumpt. Anschliessend meldet das Messgerät 120 Bereitschaft zur Probeneingabe. Wenn das Messgut mit Hilfe einer Spritze in den Durchflusskanal 24 eingegeben worden ist und den Sensor 72 erreicht hat, wird das Ende des Eingabevorgangs durch das Messgerät 120 signalisiert. Nach einer Wartezeit, in der die Elektroden des Elektrodenblocks 22 einschwingen können, beginnt der Messvorgang.

Nach Beendigung der Probenmessung wird die Pumpe 34 wieder gestartet und das Luftventil 46 geöffnet, so dass die Probe in den Druckausgleichsbehälter 146 und von dort in den Abfallbehälter 62 gepumpt wird.

Durch alternierendes Öffnen und Schliessen des Luftventils 46 und des Ventils 44 für die Zugabe der ersten Kalibrierlösung werden Flüssigkeitssegmente durch den Elektrodenkanal geschoben. Dabei sorgt die grosse Oberflächenspannung an den Luft-/Flüssigkeitsübergängen für eine gute Reinigung des Messkanals, also des Durchflusskanals 24. Weiterhin reduziert die Trennung der Flüssigkeitssegmente durch die dazwischenliegende Luft den Flüssigkeitsaustausch zwischen den Segmenten erheblich. Dieses Verfahren ermöglicht eine gute Reinigung und geringe Probenverschleppung bei geringem Elektrolytverbrauch und niedriger Dosiergeschwindigkeit.

Nach der Spülung des Durchflusskanals 24 wird ein grösseres Flüssigkeitssegment aus einem der beiden Behälter 50, 52 durch entsprechendes Öffnen der Ventile 44 bzw. 48 für die Messung eingeführt und so positioniert, dass die beiden Sensoren 70 und 72 – wie nachstehend erläutert – Lösungen melden, also ein entsprechendes Signal abgeben.

Nach dem ersten Kalibriervorgang erfolgt dann die Kalibrierung mit der zweiten Kalibrierlösung, wobei jeweils als Zwischenschritt ein Spülvorgang erfolgt. Durch Öffnen des Luftventils 46 und Pumpen mit der Pumpe 34 wird die Lösung aus dem Durchflusskanal 24 abgepumpt. Die zweite Kalibrierlösung selbst bleibt zur Benetzung der Elektroden solange im Messgerät 120, bis eine neue Messlösung zugeführt wird.

Wie bereits vorstehend erwähnt, werden zur sicheren Erkennung und Positionierung der Flüssigkeitssäulen bei den Messgeräten 20 und 120 Sensoren 70–76 verwendet, die vorteilhafterweise als IR-Lichtschranken ausgebildet sind.

In Fig. 3 ist ein Schnitt durch den Sensor 74 gemäss Fig. 1 entlang der Linie III–III gezeigt.

Dieser Sensor 74 besteht im wesentlichen aus einer Photodiode 160, der gegenüber ein Phototransistor 162 angeordnet ist. Sowohl die Photodiode 160 als auch der Phototransistor 162 sind in einem Lichtschrankengehäuse 164 angeordnet, das eine Ausnehmung 166 aufweist, in der eine zweigeteilte Schlauchführung 168 angeordnet ist. Dabei nehmen die Seitenwände der Ausnehmung 166 die Photodiode 160 und den Phototransistor 162 auf.

Die Schlauchführung weist eine Bohrung 170 zur Aufnahme des Schlauchs 172 auf, der der Leitung 30 entspricht.

Die Schlauchführung 168 sowie der Schlauch 172 sind für IR-Licht transparent, so dass eine dauerhafte Überwachung gesichert ist.

Durch die Lichtschrankenüberwachung können Fehler beim Flüssigkeitstransport, wie fehlerhafte Probeneingabe, leere Behälter für die Kalibrierlösungen, Kleben der Ventile, Luftblasen, defekte Pumpschläuche usw. sicher erkannt werden. Dies wiederum ist eine Voraussetzung für einen verdeckten Einbau der Flüssigkeitsbehälter, der Pumpe und des Schlauchsystems, wie er für ein transportables «Bedside»-Gerät unumgänglich ist.

Die Lichtschranken besitzen einen nahezu punktförmigen Sender und Empfänger. Durch geeignetes Einbringen eines transparenten Silicon-Schlauches oder einer Glaskapillare in den Strahlengang, wie in Fig. 3 gezeigt, erhält man deutlich unterschiedliche Ausgangssignale für Blut, eine wässrige Elektrolytlösung oder Luft. So wird bei Blut ein Grossteil des Lichts absorbiert. Bei einer Elektrolytlösung hingegen wirkt die Kapillare (Schlauch) ähnlich einer Zylinderlinse und fokussiert das Licht auf den Rezeptor. Dieser ist als Phototransistor ausgebildet und wird bei Blut also nur schwach angesteuert, bei wässrigen Lösungen stark und bei Luft in einem mittleren Bereich. Diese Eigenschaft kann zur Unterscheidung der Proben genutzt werden und zur Einschaltung spezieller Messprogramme für Vollblut einerseits und Serum, Plasma, Elektrolytlösung andererseits dienen.

In Fig. 4–10 sind spezielle Ausführungsformen des Elektrodenblocks und der in diesem Elektrodenblock enthaltenen Elektroden gezeigt.

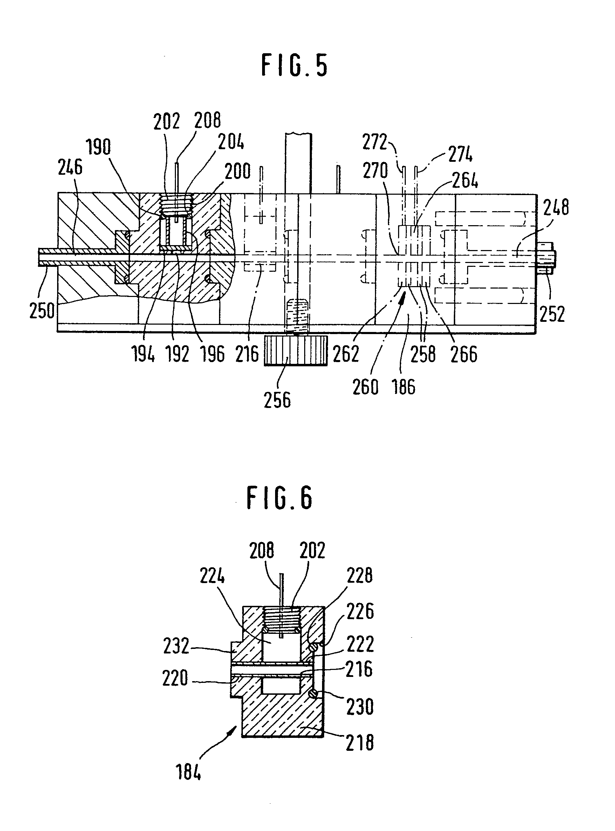

Fig. 4 und 5 zeigen einen Elektrodenblock 22 in der Draufsicht bzw. der Seitenansicht, wobei die Einzel-Elektroden schematisch gezeigt sind.

Fig. 6 zeigt eine Einzelelektrode in der Seitenansicht.

Wie aus Fig. 4 und 5 ersichtlich ist, weist der Elektrodenblock 122 vier Elektroden 180–186 auf, wobei die Elektrode 180 speziell näher erläutert ist und auch in Fig. 5 als Einzelelektrode dargestellt ist.

Jede dieser Elektroden 180–186 weist eine Querbohrung 188 auf, die den Durchflusskanal 24 bildet. Im zusammengesetzten Zustand fluchten die einzelnen Querbohrungen 188 der Elektroden 180–186 und bilden somit den Durchflusskanal 24.

Weiterhin weist jede Elektrode 180–186 senkrecht zur Achse der Querbohrung 188 eine Bohrung 190 auf, die bis nahe zur Querbohrung 188 führt. Um einen Kontakt mit dieser Querbohrung 188 herzustellen, ist eine konzentrische, jedoch engere Bohrung 192 in der Bohrung 190 vorgesehen.

Der Bodenbereich der Bohrung 190 wird im wesentlichen vollständig von einer flüssigkeitsundurchlässigen Membran 194 abgedeckt, die vorteilhafterweise als Carrier-Membran für eine ionenselektive Substanz dient.

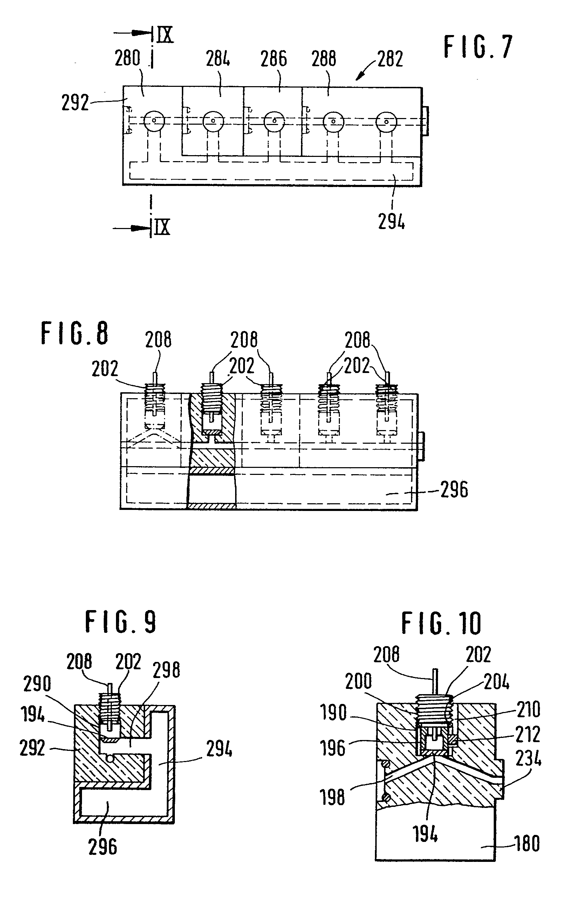

An diese Membran 194 schliesst sich ein Halteelement 196 in Form eines Einsatzes an, das in Fig. 5 und Fig. 10 gezeigt ist. Aus dieser Fig. 10 ist ebenfalls ersichtlich, dass die Querbohrung 188 gemäss Fig. 5 auch gewinkelt in Form einer gewinkelten Querbohrung 198 ausgeführt sein kann. Eine derartige v-förmige Führung hat den Vorteil, dass die Membran 194 günstiger angeströmt wird, da der Absatz, der durch die Bohrung 192 gemäss Fig. 5 gebildet wird, hierdurch entfallen kann.

An ihrem oberen Rand weist die Bohrung 190 ein Gewinde 200 auf, in das ein Stopfen 202 eingeschraubt ist, der das Halteelement 196 auf die Membran 194 drückt und diese lagefixiert.

Vorteilhafterweise ist der Stopfen 202 auf seiner der Bohrung 190 zugewandten Seite mit einer umlaufenden Ringnut 204 versehen, in die ein O-Ring eingesetzt ist, der bei eingeschraubtem Stopfen 202 dichtend an der Wand der Bohrung 190 anliegt und somit ein Herausströmen von Elektrolytflüssigkeit verhindert.

Dieser Stopfen 202 wird von dem Ableitstift 208 aus Ag/AgCl durchsetzt, der ebenfalls durch das Halteelement 196 hindurchragt und mit der Elektrolytflüssigkeit im elektrischen Kontakt steht.

Vorteilhafterweise weist die Bohrung 190 eine Längsnut 210 auf, die ein Führungselement 212 aufnimmt, das fest mit dem Halteelement 196 verbunden ist und somit ein Verdrehen des Halteelements 196 beim Einschrauben des Stopfens 202 verhindert.

Anstelle einer flächigen Ausführung der Membran 194 kann jedoch auch eine röhrenförmige Membran 216 eingesetzt sein, die vorteilhafterweise als ionenselektives Glas ausgeführt ist. Diese Ausführungsform ist speziell in Fig. 6 bei der Elektrode 184 gezeigt. Gemäss dieser Ausführungsform erstreckt sich die röhrenförmige Elektrode 216 durch den Elektrodenkörper 218, der hierzu passende Querbohrungen 220 und 222 aufweist, in die die röhrenförmige Elektrode vorteilhafterweise eingeklebt ist. Mit seinem Aussenumfang steht die röhrenförmige Elektrode im Bereich der im rechten Winkel hierzu vorgesehenen Bohrung 224 in Verbindung, die im wesentlichen der Bohrung 190 gemäss Fig. 10 gleicht, jedoch weiter unter Ausbildung eines Sacklochs nach unten gezogen ist, so dass die röhrenförmige Elektrode 216 völlig von Elektrolytflüssigkeit umspült wird. In ähnlicher Weise ist die Bohrung 224 wiederum mit einem Stopfen 202 mit Ableitstift 208 verschlossen.

Um einen flüssigkeitsdichten Elektrodenblock 22 herzustellen, weisen die Elektroden 180–186 vorteilhafterweise auf der einen Seite jeweils eine konzentrisch zur Querbohrung 188 bzw. 220, 222 verlaufende Bohrung 226 auf, die in Form eines Sacklochs ausgestaltet ist. Diese Bohrung 226 weist aus Zwecken der Abdichtung eine Ringnut 228 im Bodenbereich auf, in die ein O-Ring 230 eingelegt ist.

Die gegenüberliegende Seite weist eine zylinderförmige Erhebung 232 auf, die formschlüssig mit der Bohrung 226 einer anderen Elektrode zusammenpasst und beim Zusammenbau den O-Ring 230 staucht, wobei die gesamte Anordnung abgedichtet wird, wie dies in Fig. 4 und 5 zu sehen ist.

Um die Elektroden 180–186 lagezufixieren, sind sie in eine Halterung 234 eingespannt, die aus einer Backe 236 und einer weiteren Backe 238 besteht, die durch eine Spannschraube 240 zusammengeschraubt werden können. Die Spannschraube verbindet dabei die Backe 238 mit einem an der Hinterseite der Elektroden 180–186 vorbeigeführten Schenkel der Backe 236. In entsprechender Weise weisen diese Backen 236 und 238 eine Ausnehmung 242 und eine zylinderförmige Erhebung auf, die der Bohrung 226 bzw. der Erhebung 212 der Elektroden gemäss Fig. 6 und 10 entsprechen.

Weiterhin sind diese Backen jeweils mit einer Querbohrung 246 und 248 durchbohrt, die mit der Querbohrung 24 des Elektrodenblocks fluchten und somit eine Durchflussanordnung bilden. Weiterhin sind diese Backen auf ihrer Aussenseite jeweils mit einem Anschlussstutzen 250 und 252 versehen, an die entsprechende Schläuche angeschlossen werden können, beispielsweise die Schläuche für die Leitungen 30 und 32.

Weiterhin kann die Spannbacke 236 eine vertikale Bohrung 254 aufweisen, in die ein Führungsstift 256 eingesetzt werden kann, mit dem der Elektrodenblock an einem nicht gezeigten Gehäuse festgeschraubt wird.

Des weiteren kann die erfindungsgemässe Messvorrichtung eine Leitfähigkeitsmesszelle aufweisen, die schematisch in Fig. 5 bei der Elektrode 186 gezeigt ist. Diese weist als Leitfähigkeitsmesseinheit 260 drei Graphitscheiben 262, 264 und 266 auf, die jeweils durch eine Dichtungsscheibe 258 voneinander getrennt sind. Sämtliche Scheiben, d.h. die LF-Messeinheit 260, sind wiederum mit einer Querbohrung 270 durchbohrt, die mit dem Messkanal fluchtet. Diese Querbohrung 270 steht jeweils unmittelbar mit der LF-Messeinheit in Verbindung.

Von den äusseren Graphitscheiben 262 und 266 geht eine gemeinsame Leitung 272 ab, während die mittlere Graphitscheibe 264 mit der Leitung 274 verbunden ist. Die beiden Leitungen 272 und 274 sind mit dem Elektrodenanschlussgerät 94, beispielsweise in Form der Leitung 92, verbunden.

Die Messung der Leitfähigkeit erfolgt in üblicher Weise mittels der Messung des Wechselstromwiderstandes bei einer Frequenz von 1–10, vorzugsweise etwa 3 kHz.

Sämtliche Elektroden 180–186 im Elektrodenblock 22 bestehen vorteilhafterweise aus transparentem Kunststoffglas, beispielsweise aus Polyacrylglas, und ermöglichen eine lückenlose Kontrolle der Messkapillaren, also des Durchflusskanals 24, der vorteilhafterweise einen Durchmesser von etwa 1 mm aufweist.

Zum Schutz der hochohmigen Elektroden 180–186 vor elektrostatischen Feldern ist der Elektrodenblock 22 bis auf die Vorderseite vorteilhafterweise in Aluminium gefasst, was im Beispielsfall durch die Halteeinrichtung 234 vollzogen wird.

Die Vorderseite ist vorteilhafterweise transparent und mit einer elektrisch leitenden Auflage, beispielsweise einer InO-Auflage, beschichtet.

Die einheitliche Grösse der Elektrodenkörper 186 ermöglicht einen leichten Austausch und eine beliebige Konfiguration der Elektroden, beispielsweise kann eine Na-, K-sensitive oder eine LF-Elektrode nebeneinander angeordnet sein. Andererseits kann auch die Konfiguration folgendermassen lauten:

Na,Na,Na oder K,pH,Na.

Die kompakte Ausführung der Elektroden 180–186 unter Verzicht auf irgendwie geartete Schlauchanschlussstutzen ermöglicht einen kurzen Messkanal, so dass selbst bei drei gleichzeitig zu messenden Parametern die Probenmenge klein und die Verstopfungssicherheit durch Verwendung einer relativ weiten Kapillare als Durchflusskanal 24 gross bleibt. Die Wahl der Elektroden wird von dem Einsatzzweck her bestimmt und ist an sich unkritisch. Es können also die üblichen ionenselektiven Elektroden, beispielsweise Glaselektroden, Kunststoffelektroden aus PVC, die ein ionenselektives Material, beispielsweise Valinomycin, aufweisen, u. dgl. eingesetzt werden. Die Bohrungen 190 und 224 sind mit den üblichen Elektrolytlösungen, beispielsweise Natrium- und/oder Kaliumchlorid-Lösungen, gefüllt.

In den Fig. 7–10 ist die spezielle Ausführungsform einer Referenzelektrode 280 in Verbindung mit einem Elektrodenblock 282 gezeigt. Dieser Elektrodenblock weist neben der Referenzelektrode 280 mehrere Elektroden 284–288 auf, die in Form und Anordnung den vorher beschriebenen Elektroden 180–186 entsprechen, so dass hierauf Bezug genommen wird.

Lediglich die Referenzelektrode 280 ist speziell ausgebildet, was insbesondere aus den Fig. 7–9 ersichtlich ist. Die Referenzelektrode weist wiederum eine Vertikalbohrung 290 auf, die sich in den quaderförmigen Elektrodenkörper 292 erstreckt. An diesen Elektrodenkörper 292 schliesst sich ein die Vorderseite der Elektroden 284–288 abdeckender Hohlschenkel 294 an, der in Strömungsverbindung steht mit einem Hohlschenkel 296, der rechtwinklig abgebogen in der Horizontalen angeordnet ist und sämtliche Elektroden untergreift. Der Hohlschenkel 294 steht wiederum über einen Strömungskanal 298 mit der Bohrung 290 in Strömungsverbindung, mit der Folge, dass sämtliche Hohlschenkel 294 und 296 durch die Bohrung 290 mit der Referenzelektrolytlösung gefüllt werden können.

Diese Referenzelektrode entspricht ansonsten in ihrer Struktur der Elektrode gem. Fig. 10, wobei die Membran 194 so ausgebildet ist, dass sie möglichst geringe Mengen an Flüssigkeit in beiden Richtungen durchlässt.

Somit umschliessen also die elektrolytgefüllten Platten oder Hohlschenkel 294 und 296 die Messelektroden 284–288 vorne, und unten und durch die gute elektrische Leitfähigkeit des Innenelektrolyten werden die hochohmigen, störempfindlichen Messelektroden 284–288 von externen elektrischen Feldern abgeschirmt bis zu einer Grenzfrequenz, ab der keine ionalen Verschiebungen mehr beobachtet werden können, die folglich auch nicht mehr stören können.

Eine derartige geschlossene Referenzelektrode 280 hat nur eine maximale Lebensdauer von ca. 6 Wochen bis etwa 6 Monate. Die Begrenzung der Lebensdauer hat seine Ursache in der Kontamination der Membran 194 mit Störionen und Ablagerungen, in der abnehmenden Konzentration des Innenelektrolyten durch den Austausch über die Membran 194 und in der Verschlechterung der Eigenschaften des AgCl-Kontakts im Ableitstift 208. Die in der Abb. 7–10 gezeigte Referenzelektrode 280 lässt sich jedoch, obwohl sie relativ kompliziert aufgebaut ist, sehr gut warten, da die Verschleissteile leicht auszutauschen sind, während der Elektrodenkörper 292 weiterbenutzt werden kann. Die Membran 194 kann dadurch ausgetauscht werden, dass der Stopfen 202 ausgeschraubt und das Halte- oder Einsatzelement 196 entnommen wird. Die Membran 194 liegt dann auf der Sitzfläche frei und kann entnommen und durch eine neue Membran ersetzt werden. Anschliessend wird das Halteelement 196 und der Stopfen 202 wieder eingesetzt, wobei letzterer durch Einschrauben das Halteelement 196 und somit die Membran 194 lagefixiert. In ähnlicher Weise kann die Innenelektrolytlösung ausgetauscht werden, indem der Stopfen 202 entfernt wird und die Flüssigkeit durch eine Injektionsnadel entnommen und durch eine frische Flüssigkeit ersetzt wird.

**Patentansprüche**

1. Messvorrichtung zur Bestimmung der Aktivität oder der Konzentration von Ionen in Lösungen, mit wenigstens einer Messelektrode und einer Referenzelektrode, die mit einem Durchflusskanal (24) miteinander verbunden sind, dessen erster Ausgang (28) mit der Umgebung in Verbindung steht und dessen zweiter Ausgang (26) durch eine erste Leitung (30), in die eine erste Pumpe (34) und wenigstens ein Ventil (44, 46, 48) eingeschaltet sind, mit wenigstens einem Kalibrierlösungsbehälter (50, 52) verbunden ist, dadurch gekennzeichnet, dass von der ersten Leitung (30) zwischen der ersten Pumpe (34) und dem Durchflusskanal (24) eine Abflussleitung (54) abzweigt, in die eine zweite Pumpe und ein Absperrorgan (58) eingeschaltet sind, dass der erste Ausgang (28) des Durchflusskanals (24) mit einer Probenzuführungsleitung (32) versehen ist und dass Sensoren (70, 72) jeweils im Bereich des Ausgangs des Durchflusskanals (24) vorgesehen sind, mit denen die Füllung des Durchflusskanals (24) steuerbar ist.

2. Messvorrichtung zur Bestimmung der Aktivität oder der Konzentration von Ionen in Lösungen, mit wengistens einer Messelektrode und einer Referenzelektrode, die mit einem Durchflusskanal (24) miteinander verbunden sind, dessen erster Ausgang (28) mit der Umgebung in Verbindung steht und dessen zweiter Ausgang (26) durch eine erste Leitung (30), in die eine erste Pumpe (34) und wenigstens ein Ventil (44, 46, 48) eingeschaltet ist, mit wenigstens einem Kalibrierlösungsbehälter (50, 52) verbunden ist, dadurch gekennzeichnet, dass am ersten Ausgang (28) ein Druckausgleichsgefäss (146) vorgesehen ist, in das eine mit einer weiteren Pumpe versehene Absaugleitung (148) eintaucht, dass der zweite Ausgang (26) zusätzlich mit einer Probenzuführung (126, 136) versehen ist, und dass Sensoren (70, 72) jeweils im Bereich des Ausgangs des Durchflusskanals (24) vorgesehen sind, mit denen die Füllung des Durchflusskanals (24) steuerbar ist.

3. Messvorrichtung nach Anspruch 1 oder 2, dadurch gekennzeichnet, dass die in der Abflussleitung (54) oder der Absaugleitung (148) vorgesehene Pumpe wenigstens die gleiche Förderrate wie die in der ersten Leitung (30) vorgesehene Pumpe (34) aufweist.

4. Messvorrichtung nach Anspruch 1 oder 2, dadurch gekennzeichnet, dass die weitere Pumpe und die in der ersten Leitung (30) vorgesehene Pumpe durch eine peristaltische Schlauchpumpe gebildet sind, in deren Segmente die erste Leitung (30) und die Abflussleitung (54) oder die Absaugleitung (148) gelegt sind.

5. Messvorrichtung nach Anspruch 1 oder 2, dadurch gekennzeichnet, dass stromauf und stromab benachbart zum Durchflusskanal (24) jeweils einer der Sensoren (70, 72) und an der ersten Leitung (30) ein weiterer Sensor (74) vorgesehen sind.

6. Messvorrichtung nach Anspruch 1 oder 2, dadurch gekennzeichnet, dass an der Abflussleitung (54) oder der Absaugleitung (148) ein Sensor (76) vorgesehen ist, mit dem der Füllstand des Abfallbehälters (62) feststellbar ist.

7. Messvorrichtung nach Anspruch 1, dadurch gekennzeichnet, dass an dem dem Durchflusskanal abgewandten Ende der Probenzuführungsleitung (32) ein Adapter (64) vorgesehen ist, in dem eine auswechselbare Aspirationskanüle (66) vorgesehen ist.

8. Messvorrichtung nach Anspruch 2, dadurch gekennzeichnet, dass die erste Leitung (30) im wesentlichen senkrecht in den Durchflusskanal (24) mündet und an der Mündungsstelle sich der Durchflusskanal (24) in einen in einem Stopfen (132) vorgesehenen Kanal (136) weiter in der gleichen Achse erstreckt, wobei zwischen dem Stopfen (132) und dem Endbereich des Durchflusskanals (24) ein durchstechbares Septum (126) als Probenzuführung angeordnet ist.

9. Messvorrichtung nach Anspruch 1 oder 2, dadurch gekennzeichnet, dass sich die erste Leitung (30) stromauf der Pumpe (34) in die Leitungen (38, 40, 42) bei dem Verzeigungspunkt (36) verzweigt, in die Leitung (38, 40, 42) je ein Absperrorgan (44, 46, 48) eingeschaltet ist und die Leitungen (38) und (42) jeweils in einen Behälter (50, 52) für eine Kalibrierlösung münden, und die Leitung (40) mit der Umgebung verbunden ist.

10. Messvorrichtung nach Anspruch 5 oder 6, dadurch gekennzeichnet, dass die Sensoren (70, 72, 74, 76) elektrooptische Sensoren sind, die jeweils durch eine Photodiode (160) und einen Phototransistor (162) gebildet sind, in einem Lichtschrankengehäuse (164) angeordnet sind und ein Schlauchführungselement (168) durchstrahlen.

11. Messvorrichtung nach Anspruch 1 oder 2, dadurch gekennzeichnet, dass der Durchflusskanal (24) in einem Elektrodenblock (22) vorgesehen ist, der Elektrodenelemente (180, 182, 184, 186) aufweist, die jeweils konzentrisch zum Durchflusskanal (24) auf der einen Seite eine zylinderförmige Erhebung (232) und auf der anderen Seite eine Bohrung (226) aufweisen, die formschlüssig mit der zylinderförmigen Erhebung (232) zusammenwirkt.

12. Messvorrichtung nach Anspruch 1, dadurch gekennzeichnet, dass die Bohrung (226) eine Ringnut (228) zur Aufnahme eines O-Rings (230) zum Abdichten aufweist, und dass der Elektrodenblock (22) in einer Halterung (234) eingespannt ist, die aus einer ersten und einer zweiten Backe (236 bzw. 238) besteht, die durch eine Spannschraube (240) gegeneinander festgelegt sind.

13. Messvorrichtung nach Anspruch 11, dadurch gekennzeichnet, dass das Elektrodenelement (184) eine Bohrung (224) aufweist, deren Achse quer zur Achse des Durchflusskanals (24) verläuft und die durch einen herausschraubbaren Stopfen (202) dicht verschlossen ist, der von einem Ableitstift (208) durchsetzt ist, wobei die Bohrung (224) vom Durchflusskanal durch eine rohrförmige oder flach ausgebildete Elektrode (216, 194) getrennt ist und die Bohrung mit einer Elektrolytflüssigkeit gefüllt ist, in die der Ableitstift (208) eintaucht.

14. Messvorrichtung nach Anspruch 11, dadurch gekennzeichnet, dass die mit Elektrolytflüssigkeit gefüllte Kammer der Referenzelektrode (280) sich unter Ausbildung eines horizontalen und vertikalen Hohlschenkels (294) und (296) auf der Unterseite und der Vorderseite der Messelektroden (284-288) erstreckt und diese Messelektroden (284-288) gegen hochohmige Störfelder abschirmt.

**Claims**

1. A measuring device for determination of the activity or of the concentration of ions in solutions, with at least one measuring electrode and a reference electrode, which are connected to each other by a flow channel (24), the first outlet (28) of which is in connection with the environment and the second outlet (26) of which is connected by a first line (30), in which a first pump (34) and at least one valve (44, 46, 48) are interposed, to at least one calibrating solution container (50, 52), characterized in that from the first line (30) between the first pump (34) and the flow channel (24) a discharge line (54) branches off that a second pump and a shut-off element (58) are interposed, that the first outlet (28) of the flow channel (24) is provided with a sample feed line (32) and that sensors (70, 72) are provided in each case in the region of the outlet of the flow channel (24), with which the filling of the flow channel (24) is controllable.

2. A measuring device for determination of the activity or of the concentration of ions in solutions, with at least one measuring electrode and a reference electrode, which are connected to each other by a flow channel (24), the first outlet (28) of which is in connection with the environment and the second outlet (26) of which is connected by a first line (30), in which a first pump (34) and at least one valve (44, 46, 48) are interposed, to at least one calibrating solution container (50, 52), characterized in that at the first outlet (28) a pressure equalization vessel (146) is provided, into which dips a suction removal line (148) provided with a further pump, that said second outlet (26) is additionally provided with a sample feed line (126, 136), and that sensors (70, 72) are provided in each case in the region of the outlet of the flow channel (24), with which the filling of the flow channel (24) is controllable.

3. A measuring devices as claimed in claim 1 or 2, characterized in that the pump provided in the discharge line (54) or in the suction removal line (148) has at least the same displacement rate as the pump (34) provided in the first line (30).

4. A measuring device as claimed in claim 1 or 2, characterized in that the further pump and the pump provided in the first line (30) are formed by a peristaltic tube pump, in the segments of which the first line (30) and the discharge line (54) or the suction removal line (148) are laid.

5. A measuring device as claimed in claim 1 or 2, characterized in that there are provided upstream and downstream along side the flow channel (24) respectively one of the sensors (70, 72), and on the first line (30) a further sensor (74).

6. A measuring device as claimed in claim 1 or 2, characterized in that there is provided on the discharge line (54) or on the suction removal line (148) a sensor (76), with which the filling level of the waste container (62) can be established.

7. A measuring device as claimed in claim 1, characterized in that an adaptor (64) is provided at the end of the sample feed line (32) turning off the flow channel in which an exchangeable aspiration cannula (66) is provided.

8. A measuring device as claimed in claim 2, characterized in that the first line (30) opens out substantially at right angles into the flow channel (24) and, at the opening point, the flow channel (24) extends further in the same axis into a channel (136) provided in a stopper (132), a pierceable septum (126) as sample feed line being arranged between the stopper (132) and the end region of the flow channel (24).

9. A measuring device as claimed in claim 1 or 2, characterized in that the first line (30) branches upstream of the pump (34) into the lines (38, 40, 42) at the branch point (36), a shut-off element (44, 46, 48) is interposed in each line (38, 40, 42) and the lines (38) and (42) open out in each case into a container (50, 52) for a calibrating solution and that the line (40) is connected to the environment.

10. A measuring device as claimed in claim 5 or 6, characterized in that the sensors (70, 72, 74, 76) are electro-optical sensors which are formed in each case by a photodiode (160) and a phototransistor (162), are arranged in a light barrier housing (164) and transilluminate a tube guide element (168).

11. A measuring device as claimed in claim 1 or 2, characterized in that the flow channel (24) is provided in an electrode block (22), which has electrode elements (180, 182, 184, 186), each of which has on the one side a cylindrical elevation (232) concentric to the flow channel (24) and on the other side a bore (226), which interacts positively with the cylindrical elevation (232).

12. A measuring device as claimed in claim 1, characterized in that the bore (226) has an annular groove (228) for receiving an O-ring (230) for sealing, and that the electrode block (22) is clamped in a holder (234), which consists of a first and a second jaw (236 and 238, respectively), which are fixed against each other by a lock screw (240).

13. A measuring device as claimed in claim 11, characterized in that the electrode element (184) has a bore (224), the axis of which extends transverse to the axis of the flow channel (24) and which is closed off tightly by an unscrewable stopper (202), through which passes a drain-off pin (208), the bore (224) being separated from the flow channel by a tubular or flat-designed electrode (216, 194) and the bore being filled with an electrolyte liquid, into which the drain-off pin (208) dips.

14. A measuring device as claimed in claim 11, characterized in that the chamber of the reference electrode (280) filled with the electrolyte liquid extends on the underside and the front side of the measuring electrodes (284–288) forming a horizontal and vertical hollow leg (294) and (296), and these measuring electrodes (284–288) screen against high-resistive interference fields.

**Revendications**

1. Dispositif de mesure pour la détermination de l'activité ou de la concentration des ions en solution, comportant au moins une électrode de mesure et une électrode de référence qui sont reliées l'une à l'autre par un canal de circulation (24) dont la première sortie (28) est en communication avec le milieu environnant et dont la deuxième sortie (26) est reliée à au moins un réservoir de solution d'étalonnage (50, 52) par une première conduite (30) sur laquelle sont disposées une première pompe (34) et au moins une soupape (44, 46, 48), caractérisé en ce qu'une conduite de décharge (54) part de la première conduite (30), entre la première pompe (34) et le canal de circulation (24), conduite de décharge dans laquelle sont disposés une deuxième pompe et un organe d'obturation (58), en ce que la première sortie (28) du canal de circulation (24) est équipée d'une conduite d'alimentation de prélèvement (32) et en ce que des capteurs (70, 72) sont prévus dans la zone de sortie du canal de circulation (24), grâce auxquels on peut régler le remplissage du canal de circulation (24).

2. Dispositif de mesure pour la détermination de l'activité ou de la concentration des ions en solution, comportant au moins une électrode de mesure et une électrode de référence qui sont reliées l'une à l'autre par un canal de circulation (24) dont la première sortie (28) est en communication avec le milieu environnant et dont la deuxième sortie (26) est reliée à au moins un réservoir de solution d'étalonnage (50, 52) par une première conduite (30) sur laquelle sont disposées une première pompe (34) et au moins une soupape (44, 46, 48), caractérisé en ce qu'il est prévu, sur la première sortie (28) un récipient d'égalisation de pression (146), dans lequel plonge une conduite d'aspiration (148) équipée d'une autre pompe, en ce que la deuxième sortie (26) est équipée en outre d'une amenée de prélèvement (126, 136) et en ce que des capteurs (70, 72) sont prévus dans la zone de sortie du canal de circulation (24) grâce auxquels on peut régler le remplissage du canal de circulation (24).

3. Dispositif de mesure selon la revendication 1 ou la revendication 2, caractérisé en ce que la pompe prévue dans la conduite de décharge (54) ou dans la conduite d'aspiration (148) présente au moins le même débit que la pompe (34) prévue dans la première conduite (30).

4. Dispositif de mesure selon la revendication 1 ou la revendication 2, caractérisé en ce que l'autre pompe et la pompe prévue dans la première conduite (30) est une pompe tubulaire péristaltique dans les segments de laquelle sont placées la première conduite (30) et la conduite de décharge (54) ou la conduite d'aspiration (148).

5. Dispositif de mesure selon la revendication 1 ou la revendication 2, caractérisé en ce que, en amont et en aval et au voisinage du canal de circulation (24), sont prévus respectivement l'un des capteurs (70, 72) et sur la première conduite (30) un autre capteur.

6. Dispositif de mesure selon la revendication 1 ou la revendication 2, caractérisé en ce qu'un capteur (76), grâce auquel on contrôle le niveau du réservoir à déchets (62), est prévu sur la conduite de décharge (54) ou sur la conduite d'aspiration (148).

7. Dispositif de mesure selon la revendication 1, caractérisé en ce que, sur l'extrémité de la conduite d'amenée du prélèvement (32) opposée au canal de circulation, il est prévu un adaptateur (64) dans lequel est montée une canule d'aspiration (66) interchangeable.

8. Dispositif de mesure selon la revendication 2, caractérisé en ce que la première conduite (30) débouche sensiblement perpendiculairement dans le canal de circulation (24) et le canal de circulation (24) se prolonge, à l'embouchure, dans un canal (136) ménagé dans un bouchon (132) dans le même axe, ce qui ménage un septum perforable (126) pour l'amenée des prélèvements entre le bouchon (132) et la zone terminale du canal de circulation (24).

9. Dispositif de mesure selon la revendication 1 ou la revendication 2, caractérisé en ce que la première conduite (30) se divise en amont de la pompe (34) en conduites (38, 40, 42) au point de ramification (36), un organe de verrouillage (44, 46, 48) étant intercalé dans chaque conduite (38, 40, 42), les conduites (38) et (42) débouchant chacune dans un réservoir (50, 52) de solution d'étalonnage et la conduite (40) étant reliée à l'environnement.

10. Dispositif de mesure selon la revendication 5 ou la revendication 6, caractérisé en ce que les capteurs (70, 72, 74, 76) sont des capteurs électro-optiques qui sont chacun formés d'une photodiode (160) et d'un phototransistor (162), qui sont disposés dans un boîtier pour barrière lumineuse (164) et émettent un rayonnement lumineux au travers d'un élément de guidage de tuyau (168).

11. Dispositif de mesure selon la revendication 1 ou la revendication 2, caractérisé en ce que le canal de circulation (24) est prévu dans un bloc électrode (22) qui comporte des éléments d'électrode (180, 182, 184, 186) qui présentent chacun, concentriquement au canal de circulation (24), sur un de ses côtés, une élévation en forme de cylindre (232) et sur l'autre côté un alésage (226) qui est en engagement positif avec l'élévation en forme de cylindre (232).

12. Dispositif de mesure selon la revendication 1, caractérisé en ce que l'alésage (226) comporte une rainure (228) pour engagement avec un anneau torique d'étanchéité (230) et en ce que le bloc-électrodes (22) est encastré dans un raccord (234) qui se compose d'une première et d'une deuxième joues (resp. 236, 238) qui sont maintenues l'une contre l'autre par une vis de serrage (240).

13. Dispositif de mesure selon la revendication 11, caractérisé en ce que l'élément électrode (184) comporte un alésage (224) dont l'axe est transversal à l'axe du canal de circulation (24) et qui est fermé de façon étanche par un bouchon dévissable (202) qui est traversé par une broche de dérivation (208), l'alésage (224) étant ainsi séparé du canal de circulation par une électrode plate ou tubulaire (216, 194) et l'alésage étant rempli d'un électrolyte liquide dans lequel plonge la broche de dérivation (208).

14. Dispositif de mesure selon la revendication 11, caractérisé en ce que la chambre remplie d'électrolyte de l'électrode de référence (280) se prolonge, par formation de branches creuses horizontale et verticale (294) et (296) sur la face inférieure et sur la face avant des électrodes de mesure (284–288) et en ce que ces électrodes de mesure (284–288) protègent contre les champs parasitaires de valeur ohmique élevée.

FIG. 1

FIG. 2

FIG.3

FIG.4

# FIG.5

# FIG.6

FIG.7

FIG.8

FIG.9

FIG.10